(19) Europäisches Patentamt
European Patent Office
Office européen des brevets

(11) EP 1 284 145 A1

(12) **EUROPÄISCHE PATENTANMELDUNG**

(43) Veröffentlichungstag:
**19.02.2003 Patentblatt 2003/08**

(21) Anmeldenummer: **02016986.8**

(22) Anmeldetag: **05.08.2002**

(51) Int Cl.[7]: **A61K 47/16**, A61K 47/08, A61K 47/20, A61K 7/00, C11B 5/00, C07C 335/12, C07C 275/24, C07C 279/08

(84) Benannte Vertragsstaaten:
**AT BE BG CH CY CZ DE DK EE ES FI FR GB GR IE IT LI LU MC NL PT SE SK TR**
Benannte Erstreckungsstaaten:
**AL LT LV MK RO SI**

(30) Priorität: **17.08.2001 DE 10140443**

(71) Anmelder: **HAARMANN & REIMER GMBH**
**37603 Holzminden (DE)**

(72) Erfinder: **Ley, Jakob Peter Dr.**
**37603 Holzminden (DE)**

(74) Vertreter: **Zobel, Manfred, Dr.**
**Bayer AG**
**Bayer Chemicals**
**Legal & Patents**
**Patents and Licensing**
**51368 Leverkusen (DE)**

(54) **2-(3,4-Dihydroxyphenyl)ethyl-substituierte Kohlensäurederivate und deren Verwendung**

(57) Die Erfindung betrifft die Verwendung von 2-(3,4-Dihydroxyphenyl)ethyl-substituierten Kohlensäurederivaten als Antioxidantien oder Radikalfänger, insbesondere die Verwendung zum Schutz von Zellen und Gewebe von Menschen vor den die Alterung beschleunigenden schädlichen Einflüssen von Radikalen und reaktiven Sauerstoffverbindungen.

EP 1 284 145 A1

## Beschreibung

Die Erfindung betrifft die Verwendung von 2-(3,4-Dihydroxyphenyl)ethyl-substituierten Kohlensäurederivaten als Antioxidantien oder Radikalfänger, insbesondere die Verwendung zum Schutz von Zellen und Geweben von Menschen vor den schädlichen Einflüssen von Radikalen und reaktiven Sauerstoffverbindungen. Die Erfindung betrifft ferner kosmetische, dermatologische, der Ernährung oder dem Genuss dienende Zubereitungen enthaltend diese 2-(3,4-Dihydroxyphenyl)ethyl-substituierten Kohlensäurederivate.

Antioxidantien sind Substanzen, die im Vergleich zu dem oxidierbaren Substrat in kleinen Konzentrationen die Oxidation signifikant verzögern oder gänzlich verhindern. Viele Antioxidantien fungieren gleichzeitig als Radikalfänger und/oder als Komplexbildner für Schwermetallionen.

Es ist erstrebenswert, Substanzen zu finden, die in physiologischen Systemen die natürlichen Abwehrmechanismen gegen freie Radikale und reaktive Sauerstoffverbindungen unterstützen oder als Schutzstoffe in Kosmetika, Pharmazeutika sowie in Nahrungs- oder Genussmitteln deren oxidationsempfindlichen Bestandteile vor Autoxidation schützen.

Die Aufgabe der vorliegenden Erfindung besteht darin, Antioxidantien mit starker spezifischer radikalfangender und/oder antioxidativer Wirkung zur Verwendung in kosmetischen und pharmazeutischen Zubereitungen und in Nahrungs- oder Genussmitteln sowie zum Schutz von Zellen und Gewebe von Säugern zu entwickeln.

Die Erfindung betrifft daher die Verwendung von 2-(3,4-Dihydroxyphenyl)ethyl-substituierten Kohlensäurederivaten der allgemeinen Formel I

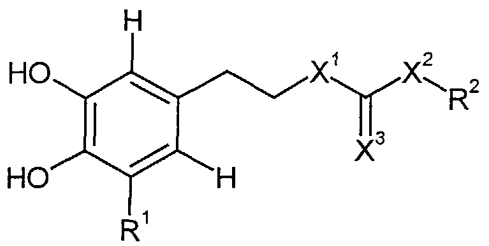

(I)

wobei

$R^1$ ein Wasserstoffatom, eine Niederalkyl-, Niederalkenyl-, 1-Oxoniederalkyl oder 1-Oxoniederalkenylgruppe oder eine Gruppe -O-$R^3$ darstellt, in der $R^3$ ein Wasserstoffatom, eine Niederalkyl-, Niederalkenyl-, 1-Oxoniederalkyl- oder 1-Oxoniederalkenylgruppe bedeutet,

und

$X^1$, $X^2$ und $X^3$ unabhängig voneinander Sauerstoff, Schwefel oder NH darstellen,

und

$R^2$ eine verzweigte oder unverzweigte, cyclische oder gestreckte Alkylgruppe mit 1 bis 22 Kohlenstoffatomen oder eine verzweigte oder unverzweigte, cyclische oder gestreckte Alkenylgruppe mit 2 bis 22 Kohlenstoffatomen, wobei eins oder mehrere der Kohlenstoffatome durch Sauerstoffatome ersetzt werden können, oder eine kernsubstituierte Arylalkylgruppe mit 7 bis 15 Kohlenstoffatomen bedeutet,

als Antioxidantien oder Radikalfänger.

Eine Niederalkylgruppe besteht bevorzugt aus 1 bis 5 Kohlenstoffatomen und kann beispielsweise sein: Methyl, Ethyl, 1-Propyl, 2-Propyl-, 1-Butyl, 2-Butyl, tert.-Butyl, 2-Methylprop-1-yl, Cyclopropyl, Cyclopropylmethyl, 2,2-Dimethylcyclopropyl, Cyclobutyl, 1-, 2- oder 3-Pentyl-, 2-Methylbut-1-yl, 2-Methylbut-2-yl, 3-Methylbut-1-yl, 3-Methylbut-2-yl oder Cyclopentyl.

Eine Niederalkenylgruppe besteht bevorzugt aus 2 bis 5 Kohlenstoffatomen und kann beispielsweise sein: Ethenyl, Prop-2-en-1-yl, Prop-1-en-1-yl, Prop-1-en-2-yl, 1- oder 2-Cyclopropenyl-, But-1-en-1-yl, But-1-en-2-yl, But-1-en-3-yl, But-2-en-1-yl, But-3-en-1-yl, But-2-en-2-yl, 2-Methylprop-1-en-1-yl, 2-Methylprop-2-en-1-yl, 1,3-Butadien-1-yl, 1,3-Butadien-2-yl, Pent-1-en-1-yl, Pent-1-en-2-yl, Pent-1-en-3-yl, Pent-1-en-4-yl, Pent-2-en-1-yl, Pent-2-en-2-yl, Pent-2-en-3-yl, Pent-2-en-4-yl, Pent-3-en-1-yl, Pent-4-en-1-yl, 1,3-Pentadien-1-yl, 1,3-Pentadien-2-yl, 1,3-Pentadien-3-yl, 2,4-Pentadien-2-yl, 2,4-Pentadien-1-yl, 1,4-Pentadien-1-yl, 1,4-Pentadien-2-yl, 1,4-Pentadien-3-yl, 1-,2- oder 3-Cyclopentenyl, 1-,2- oder 3-Cyclopentadienyl, 3-Methylbut-1-en-1-yl, 3-Methylbut-1-en-2-yl, 3-Methylbut-1-en-3-yl, 3-Methylbut-1-en-4-yl, 3-Methylbut-2-en-1-yl, 3-Methylbut-2-en-2-yl, 3-Methylbut-2-en-4-yl, 2-Methylbut-1-en-1-yl, 2-Methylbut-1-en-3-yl, 2-Methylbut-1-en-4-yl, 2-Methylidenbut-1-yl, 2-Methyl-1,3-butadien-1-yl, 2-Methyl-1,3-butadien-3-yl, 2-Methyl-1,3-butadien-4-yl, 2-Methylidenbut-3-en-1-yl, und die jeweiligen gegebenenfalls möglichen Z- und E-Isomere der vorgenannten Reste.

Eine Niederalkyl-1-oxogruppe besteht bevorzugt aus 1 bis 5 Kohlenstoffatomen und kann beispielsweise sein: Formyl, Acetyl, Propanoyl, Butanoyl, 2-Methylpropanoyl, Pentanoyl, 2- oder 3-Methylbutanoyl, 2,2-Dimethylpropanoyl oder Cyclopropylcarboxyl.

Eine 1-Oxoalkenylgruppe kann bevorzugt 3 bis 5 Kohlenstoffatome enthalten und kann beispielsweise sein: Prop-2-enoyl, 2-Methylprop-2-enoyl, 2-Ethyl-prop-2-enoyl, E- oder Z- 2-Butenoyl, 3-Butenoyl, E- oder Z- 2-Methylbut-2-enoyl, , E- oder Z- 3-Methylbut-2-enoyl, Z- oder E- 2-Pentenoyl, Z- oder E- 3-Pentenoyl.

Eine unverzweigte, verzweigte oder cyclische Alkylgruppe kann 1 bis 22, bevorzugt 1 bis 20, insbesondere bevorzugt 1 bis 18 Kohlenstoffatome enthalten. Beispielsweise seien genannt: Methyl, Ethyl, 1-Propyl, 2-Propyl-, 1-Butyl, 2-Butyl, tert.-Butyl, 2-Methyl, 2-Methylprop-1-yl, Cyclopropyl, Cyclopropylmethyl, 2,2-Dimethylcyclopropyl, Cyclobutyl, Cyclopentyl, Cyclohexyl und verschiedene Stellungsisomere von Methylpentyl, Menthyl, 1-Pentyl, 1-Hexyl, 1-Heptyl-, 1-Octyl, 2-Ethylhexyl, 1-Nonyl, 1-Decyl, 1-Undecyl, 1-Dodecyl, 1-Tridecyl, 1-Tetradecyl, 1-Pentadecyl, 1-Hexadecyl, 1-Heptadecyl und 1-Octadecyl.

Eine unverzweigte, verzweigte oder cyclische Alkenylgruppe kann 2 bis 22, bevorzugt 2 bis 20, insbesondere bevorzugt 2 bis 18 Kohlenstoffatome enthalten. Beispielsweise seien genannt: Ethenyl, Prop-2-en-1-yl, Prop-1-en-1-yl, Prop-1-en-2-yl, 1- oder 2-Cyclopropenyl-, But-1-en-1-yl, But-1-en-2-yl, But-1-en-3-yl, But-2-en-1-yl, But-3-en-1-yl, But-2-en-2-yl, 2-Methylprop-1-en-1-yl, 2-Methylprop-2-en-1-yl, 1,3-Butadien-1-yl, 1,3-Butadien-2-yl, Pent-1-en-1-yl, Pent-1-en-2-yl, Pent-1-en-3-yl, Pent-1-en-4-yl, Pent-2-en-1-yl, Pent-2-en-2-yl, Pent-2-en-3-yl, Pent-2-en-4-yl, Pent-3-en-1-yl, Pent-4-en-1-yl, 1,3-Pentadien-1-yl, 1,3-Pentadien-2-yl, 1,3-Pentadien-3-yl, 2,4-Pentadien-2-yl, 2,4-Pentadien-1-yl, 1,4-Pentadien-1-yl, 1,4-Pentadien-2-yl, 1,4-Pentadien-3-yl, 1-,2- oder 3-Cyclopentenyl, 1-,2- oder 3-Cyclopentadienyl, 3-Methylbut-1-en-1-yl, 3-Methylbut-1-en-2-yl, 3-Methylbut-1-en-3-yl, 3-Methylbut-1-en-4-yl, 3-Methylbut-2-en-1-yl, 3-Methylbut-2-en-2-yl, 3-Methylbut-2-en-4-yl, 2-Methylbut-1-en-1-yl, 2-Methylbut-1-en-3-yl, 2-Methylbut-1-en-4-yl, 2-Methylidenbut-1-yl, 2-Methyl-1,3-butadien-1-yl, 2-Methyl-1,3-butadien-3-yl, 2-Methyl-1,3-butadien-4-yl, 2-Methylidenbut-3-en-1-yl, die verschiedenen Stellungs- und Doppelbindungsisomeren des Heptenyl-, des Octenyl-, des Nonenyl-, des Decenyl-, des Dodecenyl, des Tetradecenyl-, des Hexadecenyl-, des Octadecenylrests, Z-Hexadeca-9-en-1-yl, Z-Octadeca-9-en-1-yl, Z,Z-Octadeca-9,12-dien-1-yl, Z,Z,Z-Octadeca-9,12,15-trien-1-yl und E-Octadeca-9-en-1-yl.

Eine kernsubstituierte Arylalkylgruppe kann aus 7 bis 15 Kohlenstoffatomen, bevorzugt aus 7 bis 8 Kohlenstoffatomen bestehen, mit der Maßgabe, dass mindestens ein weiterer Substituent am aromatischen Teil nicht Wasserstoff ist. Insbesondere bevorzugt ist eine kernsubstituierte Benzyl, eine 2- oder 1-Phenylethylgruppe.

Bevorzugte Substituenten der kernsubstituierten Arylalkylgruppe sind: Wasserstoffatome, Niederalkyl-, Hydroxy-, Niederalkyloxy-, Thio-, Niederalkylthio-, Amino-, Niederalkylamino-, Diniederalkylamino-, Phosphat-, Niederalkylphosphat-, Diniederalkylphosphat-, Sulfonsäure-, Niederalkylsulfonat-, Sulfonamid-, Diniederalkylsulfonamid- oder Niederalkylsulfonamidreste. Insbesondere bevorzugt sind Wasserstoffatome, Niederalkyl-, Hydroxy- und Niederalkyloxyreste.

Bevorzugt ist die Verwendung von 2-(3,4-Dihydroxyphenyl)ethyl-substituierten Kohlensäurederivaten der allgemeinen Formel I,
wobei

$R^1$ ein Wasserstoffatom, eine Niederalkylgruppe mit 1 bis 5 Kohlenstoffatomen oder eine Gruppe -O-$R^3$ darstellt, in der $R^3$ ein Wasserstoffatom oder eine Methylgruppe bedeutet,

und

$X^1$, $X^2$ und $X^3$ unabhängig voneinander Sauerstoff, Schwefel oder NH darstellen,

und

$R^2$ eine verzweigte oder unverzweigte, cyclische oder gestreckte Alkylgruppe mit 1 bis 18 Kohlenstoffatomen oder eine verzweigte oder unverzweigte, cyclische oder gestreckte Alkenylgruppe mit 2 bis 20 Kohlenstoffatomen oder einen kernsubstituierten Benzyl-, kernsubstituierten 2-Phenylethyl oder kernsubstituierten 1-Phenylethylrest darstellt,

als Antioxidantien oder Radikalfänger.

Insbesondere bevorzugt ist die Verwendung von 2-(3,4-Dihydroxyphenyl)ethyl-substituierten Kohlensäurederivaten der allgemeinen Formel I,
wobei

$R^1$ ein Wasserstoffatom, eine Methoxy- oder eine Hydroxygruppe darstellt,

und

$X^1$ eine Gruppe NH darstellt,

und

$X^2$ und $X^3$ unabhängig voneinander Sauerstoff, Schwefel oder NH darstellen,

und

$R^2$ eine verzweigte oder unverzweigte, cyclische oder gestreckte Alkylgruppe mit 1 bis 18 Kohlenstoffatomen oder eine verzweigte oder unverzweigte, cyclische oder gestreckte Alkenylgruppe mit 2 bis 18 Kohlenstoffatomen darstellt,

als Antioxidantien oder Radikalfänger.

Als erfmdungsgemäße Einzelverbindungen seien beispielsweise
*N*-[2-(3,4-Dihydroxyphenyl)ethyl]-*O*-methylurethan
*N*-[2-(3,4-Dihydroxyphenyl)ethyl]-*O*-[(1*R*,3*R*,4*S*)-menthyl]urethan
*N*-[2-(3,4-Dihydroxyphenyl)ethyl]-*O*-hexylurethan
*N*-[2-(3,4-Dihydroxyphenyl)ethyl]-*O*-(2-ethylhexyl)urethan
*N*-[2-(3,4-Dihydroxyphenyl)ethyl] -*N*'-hexylthioharnstoff
*N*-[2-(3,4-Dihydroxyphenyl)ethyl]-*N*'-hexylharnstoff
genannt.

Wenn bei den erfindungsgemäßen 2-(3,4-Dihydroxyphenyl)ethyl-substituierten Kohlensäurederivaten der allgemeinen Formel I mindestens einer der Reste $X^1$, $X^2$ und $X^3$ NH darstellt, können erfindungsgemäßen Verbindungen auch in Form eines ihrer Tautomere vorliegen.

Überraschenderweise wurde nun gefunden, dass die erfmdungsgemäßen 2-(3,4-Dihydroxyphenyl)ethyl-substituierten Kohlensäurederivate sehr gute Radikalfänger und besonders starke Antioxidantien sind. Insbesondere sind die erfindungsgemäßen 2-(3,4-Dihydroxyphenyl)ethyl-substituierten Kohlensäurederivate in der Lage, die schädlichen Einflüsse freier Radikale und/oder oxidativer Prozesse in oder auf der Haut zu unterdrücken und die natürlichen antioxidativen Prozesse zu unterstützen.

Die erfindungsgemäßen 2-(3,4-Dihydroxyphenyl)ethyl-substituierten Kohlensäurederivate stellen außerdem sehr gute Antioxidantien für hochungesättigte Lipide wie beispielsweise Squalen, Lycopin, Carotine, Docosahexaensäure, Eicosapentaensäure, $\alpha$- oder $\gamma$-Linolensäure oder Linolsäure sowie für fette Öle, enthaltend (mehrfach) ungesättigte Fettsäuren, wie beispielsweise Sojaöl, Leinöl, Distelöl, Borretschsamenöl, Nachtkerzenöl, Fischöl, Olivenöl, schwarze Johannisbeerenkemöl oder Sonnenblumenöl dar. Daher können sie in der Ernährung oder dem Genuss dienenden Zubereitungen, die solche Lipide enthalten, als Antioxidantien verwendet werden. Insbesondere sind die erfindungsgemäßen 2-(3,4-Dihydroxyphenyl)ethyl-substituierten Kohlensäurederivate auch zur Stabilisierung der reinen Lipide oder fetten Öle oder Gemische derselben hervorragend geeignet.

Die erfindungsgemäßen 2-(3,4-Dihydroxyphenyl)ethyl-substituierten Kohlensäurederivate unterstützen in physiologischen Systemen die natürlichen Abwehrmechanismen gegen freie Radikale und reaktive Sauerstoffverbindungen und schützen in Kosmetika, Pharmazeutika sowie in Nahrungs- und Genussmitteln deren oxidationsempfmdliche Bestandteile vor Autoxidation oder Photooxidation.

Die erfindungsgemäßen 2-(3,4-Dihydroxyphenyl)ethyl-substituierten Kohlensäurederivate können daher in kosmetischen, dermatologischen, der Ernährung oder dem Genuss dienenden Zubereitungen zum Schutz von Zellen und Geweben von Säugern, insbesondere des Menschen, gegenüber dem schädlichen Einfluss von freien Radikalen und reaktiven Sauerstoffspezies (Oxidation und Photooxidation) verwendet werden. Selbstverständlich können die erfindungsgemäßen 3,4-Dihydroxybenzylsubstituierten Kohlensäurederivate auch in anderen Präparaten, beispielsweise pharmazeutischen Präparaten oder Zubereitungen, Zubereitungen zum Schutz oder der Beeinflussung von Pflanzen, in Zubereitungen zur Nahrungsergänzung, in Zubereitungen zur Herstellung von Nahrungs- oder Genussmitteln oder in anderen Erzeugnissen, beispielsweise oxidationsempfindlichen natürlichen oder synthetischen Polymeren

(z.B. Kautschuk, Polyolefine), als Antioxidantien oder Radikalfänger verwendet werden.

Die Menge der erfindungsgemäßen 2-(3,4-Dihydroxyphenyl)ethyl-substituierten Kohlensäurederivate in den erfindungsgemäßen Zubereitungen beträgt 0,0001 Gew.-% bis 10 Gew.-%, bevorzugt 0,001 bis 5 Gew.-%, besonders bevorzugt 0,001 Gew.-% bis 1 Gew.-%, bezogen auf das Gesamtgewicht der Präparate.

Die 2-(3,4-Dihydroxyphenyl)ethyl-substituierten Kohlensäurederivate sind teilweise bekannt. So werden in JP 60/115 558 die Synthese von *N*-2-(3,4-Dihydroxyphenyl)ethyl-O-*n*-methyl-urethan bzw. *N*-2-(3,4-Dihydroxyphenyl) ethyl-O-*n*-hexylurethan und in *New J. Chem.* **1998,** Bd. 22, Heft 2, S. 129-135 die Synthese von *N,N'*-Bis-[2-(3,4-dihydroxyphenyl)ethyl]-harnstoff beschrieben. Die erfindungsgemäßen 2-(3,4-Dihydroxyphenyl)ethyl-substituierten Kohlensäurederivate sind aber bisher weder im Zusammenhang mit einer antioxidativen und/oder radikalfangenden Wirkung noch mit deren Gebrauch in kosmetischen Anwendungen, Nahrungs- oder Genussmitteln beschrieben worden.

2-(3,4-Dihydroxyphenyl)ethyl-substituierte Kohlensäurederivate der allgemeinen Formel (II)

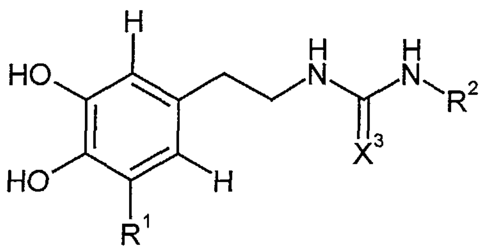

(II)

wobei

$R^1$ ein Wasserstoffatom, eine Niederalkyl-, Niederalkenyl-, 1-Oxoniederalkyl oder 1-Oxoniederalkenylgruppe oder eine Gruppe -O-$R^3$ darstellt, in der $R^3$ ein Wasserstoffatom, eine Niederalkyl-, Niederalkenyl-, 1-Oxoniederalkyl- oder 1-Oxoniederalkenylgruppe bedeutet,

und

$X^3$ Sauerstoff, Schwefel oder NH darstellt,

und

$R^2$ eine verzweigte oder unverzweigte, cyclische oder gestreckte Alkylgruppe mit 1 bis 22 Kohlenstoffatomen oder eine verzweigte oder unverzweigte, cyclische oder gestreckte Alkenylgruppe mit 2 bis 22 Kohlenstoffatomen darstellt,

sind neu.

Bevorzugt sind 2-(3,4-Dihydroxyphenyl)ethyl-substituierte Kohlensäurederivate der allgemeinen Formel (II), wobei

$R^1$ ein Wasserstoffatom, eine Niederalkylgruppe mit 1 bis 5 Kohlenstoffatomen oder eine Gruppe -O-$R^3$ darstellt, in der $R^3$ ein Wasserstoffatom oder eine Methylgruppe bedeutet,

und

$X^3$ Sauerstoff oder Schwefel darstellt,

und

$R^2$ eine verzweigte oder unverzweigte, cyclische oder gestreckte Alkylgruppe mit 1 bis 18 Kohlenstoffatomen oder eine verzweigte oder unverzweigte, cyclische oder gestreckte Alkenylgruppe mit 2 bis 20 Kohlenstoffatomen darstellt.

Als erfindungsgemäße Einzelverbindungen seien beispielsweise

*N*-[2-(3,4-Dihydroxyphenyl)ethyl]-*N'*-hexylthioharnstoff
*N*-[2-(3,4-Dihydroxyphenyl)ethyl]-*N'*-hexylharnstoff
genannt.

Die 2-(3,4-Dihydroxyphenyl)ethyl-substituierten Kohlensäurederivate der allgemeinen Formel (II) können dargestellt werden, indem man 2-(3,4-Dihydroxyphenyl)ethylamine der allgemeinen Formel (III)

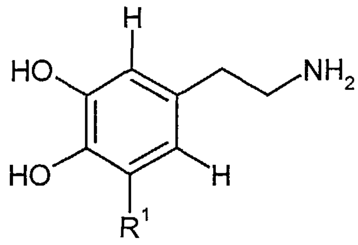

(III)

wobei $R^1$ die oben aufgeführte Bedeutung hat,
oder deren kationischen Salze
mit einem Heterokumulen der allgemeinen Formel (IV),

$$X^4{=}C{=}N{-}R^2 \qquad (IV)$$

wobei

$X^4$ ein Sauerstoffatom oder ein Schwefelatom darstellt

und

$R^2$ die oben genannte Bedeutung hat,

oder
mit Phosgen, Thiophosgen oder Triphosgen mit oder ohne Lösungsmittel und gegebenenfalls unter Beimengung einer Hilfsbase und anschließend entweder direkt nach Aufreinigung oder ohne Aufreinigung mit einer Verbindung der allgemeinen Formel (V),

$$Z{-}R^2 \qquad (V)$$

wobei

Z eine Gruppe -O-H, -S-H, $-NH_2$ oder $-(NH_3)^+$ darstellt

und

$R^2$ die oben genannte Bedeutung hat

mit oder ohne Lösungsmittel und gegebenenfalls unter Beimengung einer Hilfsbase umsetzt.

Die 2-(3,4-Dihydroxyphenyl)ethyl-substituierten Kohlensäurederivate der allgemeinen Formel (II) können bevorzugt durch Umsetzung von 2-(3,4-Dihydroxyphenyl)ethylaminen der allgemeinen Formel (III), wobei $R^1$ die oben genannte Bedeutung hat, mit einem Heterokumulen der allgemeinen Formel (IV), wobei $R^2$ die oben genannte Bedeutung hat, in einem Lösungsmittel oder Lösungsmittelgemisch, bevorzugt ausgewählt aus der Gruppe Wasser, Aceton, 1,4-Dioxan, Tetrahydrofuran, aliphatischen Estern aliphatischer Alkohole (wie z.B. Essigsäureethylester), chlorhaltigen Lösungsmitteln (z.B. Chloroform) oder aromatischen Lösungsmitteln (z.B. Benzol), und vorteilhafterweise unter Beimengung einer oder mehrerer Hilfsbasen, bevorzugt ausgewählt aus der Gruppe der Alkalimetallhydroxide (z.B. NaOH), der Alkalimetallcarbonate (z.B. $Na_2CO_3$ oder $NaHCO_3$), der Erdalkalimetallhydroxyide (z.B. $Mg(OH)_2$), der Erdalkalimetalloxide (z.B. CaO) oder der Erdalkalimetallcarbonate (z.B. $CaCO_3$), Ammoniak, der aliphatischen Amine (z.

B. Triethylamin oder Diisopropylamin) oder der heterocyclischen Amine (z.B. Pyridin oder 4-(N,N-Dimethylamino)pyridin) oder der basischen anorganischen oder organischen Ionenaustauscher, erhalten werden.

Die vorliegende Erfindung betrifft weiterhin auch kosmetische und dermatologische Zubereitungen, welche die erfindungsgemäßen 2-(3,4-Dihydroxyphenyl)ethyl-substituierten Kohlensäurederivate in einer wirksamen Menge, nebst anderen, ansonsten üblichen Zusammensetzungsbestandteilen, umfassen. Sie enthalten 0,0001 Gew.-% bis 10 Gew.-%, bevorzugt 0,001 bis 5 Gew.-%, insbesondere aber 0,001 Gew.-% bis 1 Gew.-%, bezogen auf das Gesamtgewicht der Formulierung, an den erfindungsgemäßen 2-(3,4-Dihydroxyphenyl)ethyl-substituierten Kohlensäurederivaten und können dabei als "Wasser in Öl"-, "Öl in Wasser"-, "Wasser in Öl in Wasser"- oder "Öl in Wasser in Öl"-Emulsionen, als Mikroemulsionen, als Gele, als Lösungen z.B. in Ölen, Alkoholen oder Siliconölen, als Seifen, als Stifte, als Aerosole, Sprays oder auch Schäume vorliegen. Weitere übliche kosmetische oder dermatologische Hilfsund Zusatzstoffe können in Mengen von 5 bis 99,9999 Gew.-%, vorzugsweise 10 bis 80 Gew.-%, bezogen auf das Gesamtgewicht der Formulierung, enthalten sein. Ferner können die Formulierungen Wasser in einer Menge bis zu 99,999 Gew.-%, vorzugsweise 5 bis 80 Gew.-%, bezogen auf das Gesamtgewicht der Formulierung, aufweisen.

Die vorliegende Erfindung betrifft zudem auch der Ernährung oder dem Genuss dienende Zubereitungen, welche die erfindungsgemäßen 2-(3,4-Dihydroxyphenyl)ethylsubstituierten Kohlensäurederivate in einer wirksamen Menge, nebst anderen, ansonsten üblichen Zusammensetzungsbestandteilen, umfassen. Sie enthalten 0,0001 Gew.-% bis 10 Gew.-%, bevorzugt 0,001 bis 5 Gew.-%, insbesondere aber 0,001 Gew.-% bis 1 Gew.-%, bezogen auf das Gesamtgewicht der Formulierung, an den erfindungsgemäßen 2-(3,4-Dihydroxyphenyl)ethyl-substituierten Kohlensäurederivaten und können dabei als Gele, als Lösungen z.B. in Ölen, Wasser, Ethanol, als Dispersionen, Emulsionen oder Suspensionen in Öl oder Wasser, oder auch gemischt mit Trockenmassen vorkommen. Weitere übliche der Ernährung oder dem Genuss dienende Grund-, Hilfs- und Zusatzstoffe können in Mengen von 5 bis 99,9999 Gew.-%, vorzugsweise 10 bis 80 Gew.-%, bezogen auf das Gesamtgewicht der Formulierung, enthalten sein. Ferner können die Formulierungen Wasser in einer Menge bis zu 99,999 Gew.-%, vorzugsweise 5 bis 80 Gew.-%, bezogen auf das Gesamtgewicht der Formulierung, aufweisen.

Die erfindungsgemäßen Zubereitungen, enthaltend die 2-(3,4-Dihydroxyphenyl)-ethyl-substituierten Kohlensäurederivate, werden mit üblichen, an sich bekannten Verfahren hergestellt, dergestalt, dass eins oder mehrere der erfindungsgemäßen 2-(3,4-Dihydroxyphenyl)ethyl-substituierten Kohlensäurederivate in die Formulierungen eingearbeitet werden, die wie üblich zusammengesetzt sind und zur Behandlung, dem Schutz, der Pflege und der Reinigung der Haut oder der Haare, als Schminkprodukte sowie als Nahrungs- oder Genussmittel oder als Getränk dienen können.

Zur Herstellung der erfindungsgemäßen Zubereitungen können in einer weiteren Ausführungsform die erfindungsgemäßen 2-(3,4-Dihydroxyphenyl)ethyl-substituierten Kohlensäurederivate auch vorher in Liposomen, z.B. ausgehend von Phosphatidylcholin, in Microsphären, in Nanosphären oder auch in Kapseln aus einer geeigneten Matrix, z.B. aus natürlichen oder synthetischen Wachsen, beispielsweise Bienenwachs, Carnaubawachs, Siliconwachs oder Paraffinwachse sowie Stearylalkohol, Eicosanol, Cetylalkohol, Stearin, Kohlenhydraten wie z.B. Stärke oder aus Proteinen, wie z.B. Gelatine, eingearbeitet werden. Eine weitere Ausführungsform besteht darin, dass die erfindungsgemäßen 2-(3,4-Dihydroxyphenyl)ethyl-substituierten Kohlensäurederivate vorher mit Komplexbildnern, beispielsweise mit Cyclodextrinen oder Cyclodextrinderivaten, bevorzugt Methylcyclodextrin, komplexiert werden.

Die erfindungsgemäßen kosmetischen und dermatologischen Zubereitungen können kosmetische Hilfs- und Zusatzstoffe enthalten, wie sie üblicherweise in solchen Zubereitungen vewendet werden, z.B. Sonnenschutzmittel (z. B. organische oder anorganische Lichtfiltersubstanzen, bevorzugt Mikropigmente), Konservierungsmittel, Bakterizide, Fungizide, Viruzide, Kühlwirkstoffe, Pflanzenextrakte, entzündungshemmende Wirkstoffe, die Wundheilung beschleunigende Stoffe (z.B. Chitin oder Chitosan und dessen Derivate), filmbildende Substanzen (z.B. Polyvinylpyrrolidone oder Chitosan oder dessen Derivate), gebräuchliche Antioxidantien, Vitamine (z.B. Vitamin C und Derivate, Tocopherole und Derivate, Vitamin A und Derivate), 2-Hydroxycarbonsäuren (z.B. Citronensäure, Äpfelsäure, L-, D-, oder dl-Milchsäure), Hautaufhellungsmittel (z.B. Kojisäure, Hydrochinon oder Arbutin), Hautfärbungsmittel (z.B. Walnussextrakte oder Dihydroxyaceton), Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchthaltende Substanzen (z.B. Glycerin oder Harnstoff), Fette, Öle, ungesättigte Fettsäuren oder deren Derivate (z.B. Linolsäure, $\alpha$-Linolensäure, $\gamma$-Linolensäure oder Arachidonsäure und deren jeweiligen natürlichen oder synthetischen Ester), Wachse oder andere übliche Bestandteile einer kosmetischen oder dermatologischen Formulierung wie Alkohole, Polyole, Polymere, Schaumstabilisatoren, Elektrolyte, organische Lösungsmittel, Silikonderivate oder Chelatbildner (z.B. Ethylendiamintetraessigsäure und Derivate).

Die erfindungsgemäßen der Ernährung oder dem Genuss dienenden Zubereitungen können Grund-, Hilfs- und Zusatzstoffe enthalten, wie sie üblicherweise in solchen Zubereitungen verwendet werden, z.B. Fleischerzeugnisse, Fischerzeugnisse, Getreideerzeugnisse, Gemüseerzeugnisse, Obsterzeugnisse, Kohlenhydrate, Proteine, Peptide, Aminosäuren, natürliche oder künstliche Süßstoffe, mineralische Salze, Bitterstoffe, mineralische oder organische Säuren, Geschmacksmodulierer, Konservierungsmittel, Bakterizide, Fungizide, Viruzide, Pflanzenextrakte, tierische Extrakte, gebräuchliche Antioxidantien, Vitamine (z.B. Vitamin A, die Vitamine der B-Gruppe, Vitamin C, Tocopherol),

2-Hydroxycarbonsäuren (z.B. Citronensäure, Äpfelsäure, L-, D-, oder dl-Milchsäure), Aromen, Substanzen zum Verhindern des Schäumens, Farbstoffe, Pigmente, die eine färbende Wirkung haben, Verdickungsmittel, oberflächenaktive Substanzen, Emulgatoren, Fette, Öle, ungesättigte Fettsäuren oder deren Derivate (z.B. Linolsäure, α-Linolensäure, γ-Linolensäure, Eicosapentaensäure, Docosahexaensäure oder Arachidonsäure und deren jeweiligen natürlichen Ester), Wachse oder andere übliche Bestandteile.

Die jeweils einzusetzenden Mengen können in Abhängigkeit von der Art des jeweiligen Produkts vom Fachmann durch einfaches Ausprobieren leicht ermittelt werden.

Bevorzugt können die erfindungsgemäßen Zubereitungen zusätzlich neben einem oder mehreren der erfindungsgemäßen 2-(3,4-Dihydroxyphenyl)ethyl-substituierten Kohlensäurederivate auch andere Antioxidantien enthalten. Insbesondere können als andere Antioxidantien alle für die entsprechende Anwendungen geeigneten oder gebräuchlichen Antioxidantien verwendet werden. Vorteilhaft werden die Antioxidantien ausgewählt aus der Gruppe bestehend aus Aminosäuren (z.B. Glycin, Histidin, 3,4-Dihydroxyphenylalanin, Tyrosin, Tryptophan) und deren Derivate, Imidazole (z.B. Urocaninsäure) und deren Derivate, Peptide (D,L-Carnosin, D-Carnosin, L-Carnosin, Anserin) und deren Derivate, Carotinoide, Carotine (z.B. α-Carotin, β-Carotin, Lycopin) und deren Derivate, Chlorogensäure und deren Derivate, Liponsäure und deren Derivate, Aurothioglucose, Propylthiouracil und andere Thiole (z.B. Thioredoxin, Glutathion, Cystein, Cystin, Cystamin und deren Glycosyl- und N-Acylderivate oder deren Alkylester) sowie deren Salze, Dilaurylthiodipropionat, Distearylthiodipropionat, Thiodipropionsäure und deren Derivate sowie Phenolsäureamide phenolischer Benzylamine (z.B. Homovanillinsäure-, 3,4-Dihydroxyphenylessigsäure-, Ferulasäure-, Sinapinsäure-, Kaffeesäure-, Dihydroferulasäure-, Dihydrokaffeesäure-, Vanillomandelsäure- oder 3,4-Dihydroxymandelsäureamide des 3,4-Dihydroxybenzyl-, 2,3,4-Trihydroxybenzyl- bzw. 3,4,5-Trihydroxybenzylamins), Catecholoxime oder Catecholoximether (z.B. 3,4-Dihydroxybenzaldoxim oder 3,4-Dihydroxybenzaldehyd-O-ethyloxim), ferner (Metall)chelatoren (z.B. 2-Hydroxyfettsäuren, Phytinsäure, Lactoferrin), Huminsäure, Gallensäuren, Gallenextrakte, Bilirubin, Biliverdin, Folsäure und deren Derivate, Ubichinon und Ubichinol und deren Derivate, Vitamin C und dessen Derivate (z. B. Ascorbylpalmitat, Magnesiumascorbylphosphat, Ascorbylacetat), Tocopherole und Derivate (z.B. Vitamin-E-Acetat), Vitamin A und Derivate (z.B. Vitamin-A-Palmitat), Rutinsäure und deren Derivate, Flavonoide (z.B. Quercetin, α-Glucosylrutin) und deren Derivate, Phenolsäuren (z.B. Gallussäure, Ferulasäure) und deren Derivate (z.B. Gallussäurepropylester, -ethylester, -octylester), Furfurylidenglucitol, Dibutylhydroxytoluol, Butylhydroxyanisol, Harnsäure und deren Derivate, Mannose und deren Derivate, Zink und dessen Derivate (z.B. ZnO, $ZnSO_4$), Selen und dessen Derivate (z.B. Selenomethionin), Stilbene und deren Derivate (z.B. Stilbenoxid, Resveratrol) und die erfindungsgemäß geeigneten Derivate dieser genannten Wirkstoffe.

Die Menge der weiteren Antioxidantien kann in den erfindungsgemäßen Zubereitungen im allgemeinen 0,0001 bis 30 Gew.-%, bevorzugt 0,001 bis 20 Gew.-%, besonders bevorzugt 0,001 bis 5 Gew.-%, bezogen auf das Gesamtgewicht der Zubereitung, betragen.

Außer den erfindungsgemäßen 2-(3,4-Dihydroxyphenyl)ethyl-substituierten Kohlensäurederivaten können selbstverständlich mehrere weitere Antioxidantien eingesetzt werden.

In den erfindungsgemäßen kosmetischen oder dermatologischen Zubereitungen können aber auch UV-A- und/oder UV-B-Filtersubstanzen eingesetzt werden, wobei die Gesamtmenge an Filtersubstanzen 0,1 bis 30 Gew.-%, vorzugsweise 0,5 bis 10 Gew.-%, bezogen auf das Gesamtgewicht der Zubereitungen, betragen kann, wobei man zum Beispiel Sonnenschutzmittel für Haut und Haar erhält. Als UV-Aund/oder UV-B-Filtersubstanzen können beispielsweise 3-Benzylidencampherderivate (z.B. 3-(4-Methylbenzyliden)-dl-campher), Aminobenzoesäurederivate (z.B. 4-(N,N-Dimethylamino)benzoesäure-2-ethylhexylester oder Menthylanthranilat), 4-Methoxycinnamate (z.B. 2-Ethylhexyl-p-methoxycinnamat oder Isoamyl-p-methoxycinnamat), Benzophenone (z.B. 2-Hydroxy-4-methoxybenzophenon), ein- oder mehrfach sulfonierte UV-Filter [z.B. 2-Phenylbenzimidazol-5-sulfonsäure, Sulisobenzone oder 1,4-Bis (benzimidazolyl)-benzol-4,4',6,6'-tetrasulfonsäure bzw. 3,3'-(1,4-Phenylendimethyliden)-bis-(7,7-dimethyl-2-oxo-bicyclo-[2,2,1]heptan-1-methansulfonsäure) und deren Salze], Salicylate (z.B. 2-Ethylhexylsalicylat oder Homomenthylsalicylat), Triazine {z.B. 2,4-Bis-[4-(2-ethylhexyloxy)-2-hydroxyphenyl]-6-(4-methoxyphenyl)-1,3,5-triazin, 4,4'-([6-([(1,1-dimethylethyl)-aminocarbonyl]phenylamino)-1,3,5-triazin-2,4-diyl]diimino)bisbenzoesäurebis-(2-ethylhexyl)-ester)}, 2-Cyanopropensäurederivate (z.B. 2-Ethylhexyl-2-cyano-3,3-diphenyl-2-propenoat), Dibenzoylderivate (z.B. 4-tert-Butyl-4'-methoxydibenzoylmethan), polymergebundende UV-Filter (z.B. Polymer von N-[2-(bzw. 4)-(2-Oxo-3-bornyliden)methyl]benzylacrylamid) oder Pigmente (z.B. Titandioxide, Zirkondioxide, Eisenoxide, Siliciumdioxide, Manganoxide, Aluminiumoxide, Ceroxide oder Zinkoxide) verwendet werden.

Die Lipidphase in den erfindungsgemäßen kosmetischen und/oder dermatologischen Zubereitungen kann vorteilhaft gewählt werden aus folgenden Substanzgruppen: Mineralöle (vorteilhaft Paraffinöl), Mineralwachse, Kohlenwasserstoffe (vorteilhaft Squalan oder Squalen), synthetische oder halbsynthetische Triglyceridöle (z.B. Triglyceride der Caprin- oder Caprylsäure), natürliche Öle (z.B. Rizinusöl, Olivenöl, Sonnenblumenöl, Sojaöl, Erdnussöl, Rapsöl, Mandelöl, Palmöl, Kokusöl, Palmkernöl, Borretschsamenöl und dergleichen mehr), natürliche Esteröle (z.B. Jojobaöl), synthetische Esteröle (bevorzugt Ester von gesättigten und/oder ungesättigten, linearen und/oder verzweigten Alkancarbonsäuren von 3 bis 30 C-Atomen mit gesättigten und/oder ungesättigten, linearen und/oder verzweigten Alkoholen

mit 3 bis 30 C-Atomen und Ester von aromatischen Carbonsäuren mit gesättigten und/oder ungesättigten, linearen und/oder verzweigten Alkoholen mit 3 bis 30 C-Atomen, insbesondere ausgewählt aus der Gruppe Isopropylmyristat, Isopropylstearat, Isopropylpalmitat, Isopropyloleat, n-Butylstearat, n-Hexyllaurat, n-Decyllaurat, Isooctylstearat, Isononylstearat, Isononylisononanoat, 2-Ethylhexylpalmitat, 2-Ethylhexyllaureat, 2-Hexyldecylstearat, 2-Octyldecylpalmitat, Oleyloleat, Oleylerucat, Erucyloleat, Erucylerucat sowie synthetische oder natürliche Gemische solcher Ester), Fette, Wachse und andere natürliche und synthetische Fettkörper, vorzugsweise Ester von Fettalkoholen mit Alkoholen niedriger C-Zahl (z.B. mit Isopropanol, Propylenglycol oder Glycerin) oder Ester von Fettalkoholen mit Alkansäuren niedriger C-Zahl oder mit Fettsäuren, Alkylbenzoate (z.B. Gemische von n-Dodecyl-, n-Tridecyl-, n-Tetradecyl- und n-Pentadecylbenzoat) sowie cyclische oder lineare Silikonöle (wie z.B. Dimethylpolysiloxane, Diethylpolysiloxane, Diphenylpolysiloxane sowie Mischformen daraus).

Die wässrige Phase der erfindungsgemäßen kosmetischen und/oder dermatologischen Zubereitungen enthält gegebenenfalls vorteilhaft Alkohole, Diole oder Polyole niedriger C-Zahl, sowie deren Ether, vorzugsweise Ethanol, Isopropanol, Propylenglycol, Glycerin, Ethylenglycol, Ethylenglycolmonoethyl- oder -monobutylether, Propylenglycolmonomethylether-, -monoethyl- oder -monobutylether, Diethylenglycolmonomethyl- oder monoethylether und analoge Produkte, ferner Alkohole niedriger C-Zahl, z.B. Ethanol, Isopropanol, 1,2-Propandiol, Glycerin, weiterhin $\alpha$- oder $\beta$-Hydroxysäuren, vorzugsweise Milchsäure, Zitronensäure oder Salicylsäure, daneben Emulgatoren, welche vorteilhaft ausgewählt werden können aus der Gruppe der ionischen, nichtionischen, polymeren, phosphathaltigen und zwitterionischen Emulgatoren, sowie insbesondere ein oder mehrere Verdickungsmittel, welches oder welche vorteilhaft ausgewählt werden können aus der Gruppe Siliciumdioxid, Aluminiumsilikate, wie z.B. Bentonite, Polysaccharide bzw. deren Derivate, z.B. Hyaluronsäure, Guarkernmehl, Xanthangummi, Hydroxypropylmethylcellulose oder Allulose-Derivate, besonders vorteilhaft aus der Gruppe der Polyacrylate, bevorzugt ein Polyacrylat aus der Gruppe der sogenannten Carbopole, jeweils einzeln oder in Kombination oder aus der Gruppe der Polyurethane.

Die erfindungsgemäßen der Ernährung oder dem Genuss dienender Zubereitungen können neben üblicherweise verwendeten tierischen oder pflanzlichen Rohstoffen zusätzlich Wasser, Squalan oder Squalen, natürliche Öle (z.B. Olivenöl, Sonnenblumenöl, Sojaöl, Erdnussöl, Rapsöl, Mandelöl, Palmöl, Kokosöl, Palmkernöl, Borretschsamenöl und dergleichen mehr), natürliche Esteröle (z.B. Jojobaöl), Fette, Wachse und andere natürliche Fettkörper, Kohlenhydrate, beispielsweise Glucose, Sucrose oder Lactose, Süßstoffe, beispielsweise Aspartam, Cyclamat, Saccharin, Xylit oder Sorbitol, Bitterstoffe, beispielsweise Coffein oder Chinin, bitterunterdrückende Stoffe, beispielsweise Lactisol, geschmacksverstärkende Stoffe, beispielsweise Natriumglutamat oder Inositolphosphat, Aminosäuren, beispielsweise Glycin, Alanin, Leucin, Isoleucin, Valin, Prolin, Lysin, Asparagin, Asparaginsäure, Glutamin, Glutaminsäure, Tryptophan, Phenylalanin, Tyrosin, Threonin, Serin, Cystin, Cystein, Methionin, Hydroxyprolin, Arginin oder Histidin, Peptide, Proteine, Enzyme, Fruchtsäuren, vorzugsweise Milchsäure, Äpfelsäure oder Zitronensäure, daneben Emulgatoren, welche vorteilhaft ausgewählt werden können aus der Gruppe der ionischen, nichtionischen, polymeren, phosphathaltigen und zwitterionischen Emulgatoren, sowie insbesondere ein oder mehrere Verdickungsmittel, welches oder welche vorteilhaft ausgewählt werden können aus der Gruppe der Polysaccharide bzw. deren Derivate, z.B. Hyaluronsäure, Guarkernmehl, Johannisbrotkernmehl, Xanthangummi, oder Allulose-Derivate, natürliche, naturidentische oder synthetische Aromen sowie Salze, beispielsweise Natriumchlorid oder Kaliumchlorid enthalten.

Als besonders vorteilhafte Verkörperung der vorliegenden Erfindung wird die Verwendung der erfindungsgemäßen Zubereitungen, enthaltend einen wirksamen Bestandteil an den erfindungsgemäßen 2-(3,4-Dihydroxyphenyl) ethyl-substituierten Kohlensäurederivaten, zum Schutz von Geweben und Zellen von Säugern vor oxidativer Beanspruchung und dem schädlichen Einfluss von Radikalen angesehen.

Ebenso umfasst die vorliegende Erfindung auch ein Verfahren zum Schutze kosmetischer, dermatologischer sowie der Ernährung oder dem Genuss dienender Zubereitungen gegen Oxidation oder Photooxidation, wobei es sich bei diesen Zubereitungen z.B. um Zubereitungen zur Behandlung, zum Schutz und zur Pflege der Haut, der Nägel oder der Haare oder auch um Nahrungs- und Genussmittel oder Getränke handeln kann, deren Bestandteile Stabilitätsprobleme aufgrund von Oxidation bzw. Photooxidation bei der Lagerung mit sich bringen, dadurch gekennzeichnet, dass die erfindungsgemäßen Zubereitungen einen wirksamen Gehalt an erfindungsgemäßen 2-(3,4-Dihydroxyphenyl) ethyl-substituierten Kohlensäurederivaten aufweisen.

## Beispiele

Die nachfolgenden Beispiele sollen die vorliegende Erfindung verdeutlichen, ohne sie einzuschränken.

### Synthesevorschriften

Beispiel 1: *N*-[2-(3,4-Dihydroxyphenyl)ethyl]-*O*-methylurethan)

2-(3,4-Dihydroxyphenyl)ethylaminhydrochlorid (3 g, 15,9 mmol) wird in 1,4-Dioxan und Wasser (1:1, 60 ml)

gelöst und Natriumhydrogencarbonat (1,33 g) zugegeben. In die Mischung wird Chlorameisensäuremethylester (1,5 g, 15,9 mmol) getropft. Nach Zugabe der halben Lösung wird noch einmal eine Portion Natriumhydrogencarbonat (1,33 g, insges. 15,9 mmol) zugegeben. Die Mischung wird noch 2 h bei Raumtemperatur nachgerührt, mit 5 %iger $H_2SO_4$ auf pH 1-2 gebracht, mit *tert*-Butylmethylether (3mal 50 ml) extrahiert, die vereinigten organischen Phasen mit ges. NaCl-Lsg. gewaschen, über $Na_2SO_4$ getrocknet, abfiltriert und das Filtrat bei 45°C/20 mbar eingedampft (5,7 g, gelbliches Öl). Das Rohprodukt wird in 1,4-Dioxan und Wasser (1:1, 20 ml) gelöst, ges. $NaHCO_3$-Lsg. (1 ml) zugegegeben und die Mischung bei 80°C 3 h gerührt. Die Reaktionsmischung wird mit 5 %iger $H_2SO_4$ auf ca. pH 4 gebracht, mit *tert*-Butylmethylether (3 mal 50 ml) extrahiert, die vereinigten organischen Phasen mit ges. NaCl-Lsg. gewaschen, über $Na_2SO_4$ getrocknet, abfiltriert und das Filtrat bei 45°C/20 mbar eingedampft (5,5 g). Das Produkt wird an Kieselgel 60 mit dem Eluenten $CHCl_3/CH_3OH$ 7:1 (v/v) gereinigt: 3,3 g gelbes Öl (99 % d.Th.); Reinheit 99 % (HPLC); $^1$H-NMR (400 MHz, $CD_3OD$): $\delta$ = 6,67 (1H, d, 8 Hz), 6,62 (1H, d, 2 Hz), 6,51 (1H, dd, 8 Hz, 2 Hz), 3,62 (3H, s), 3,24 (2H, t, 7 Hz), 2,62 (2H, t, 7 Hz) ppm; MS (APCI-): *m/z* = 210,35 (47 %,
$[M-H]^-$), 420,75 (100 %, $[2M-H]^-$).

Analog wurden die folgenden Verbindungen erhalten:

Beispiel 2: *N*-[2-(3,4-Dihydroxyphenyl)ethyl]-*O*-[(1*R*,3*R*,4*S*)-menthyl]urethan

Ausbeute: quant.; Reinheit 100 % (HPLC); $^1$H-NMR (200 MHz, $CD_3OD$): $\delta$ = 6,66 (1H, d, 7,5 Hz), 6,62 (1H, d, 2 Hz), 6,49 (1H, dd, 7,5 Hz, 2 Hz), 4,47 (1H, td, 11 Hz, 5 Hz), 3,23 (2H, t, 7 Hz), 2,60 (2H, t, 7 Hz), 2,03-1,83 (2H, m), 1,78-1,60 (2H, m), 1,6-0,7 (m, 14 H) ppm; MS (ESI+): *m/z* = 198,80 (100 %), 336,45 (96 %, $[M+H]^+$).

Beispiel 3: *N*-[2-(3,4-Dihydroxyphenyl)ethyl]-*O*-hexylurethan

Ausbeute 55 %; $^1$H-NMR (400 MHz, $CD_3OD$): $\delta$ = 6,67 (1H, d, 8Hz), 6,63 (1H, d, 2 Hz), 6,50 (1H, dd, 8 Hz, 2 Hz), 3,99 (2H, t, 7 Hz), 3,24 (2H, t, 7,5 Hz), 2,62 (2H, t, 7,5 Hz), 1,58 (2H, m), 1,42-1,25 (6H, m), 0,91 (3H, t, 7 Hz) ppm; Reinheit: 99 %; MS (APCI+): *m/z* = 281,99 (100 %, $[M+H]^+$).

Beispiel 4: *N*-[2-(3,4-Dihydroxyphenyl)ethyl]-*O*-(2-ethylhexyl)urethan

Ausbeute 80 %; Reinheit 100 % (HPLC); $^1$H-NMR (400 MHz, $CD_3OD$): $\delta$ = 6,68 (1H, d, 8Hz), 6,62 (1H, d, 2 Hz), 6,50 (1H, dd, (Hz, 2 Hz), 3,93 (2H, d, 7 Hz), 3,23 (2H, t, 7,5 Hz), 2,61 (2H, t, 7,5 Hz), 1,54 (1H, m), 1,42-1,26 (8H, m), 0,96-0,84 (6H, m) ppm; MS (APCI-): *m/z* = 308,27 (32 %, $[M-H]^-$), 350,70 (100 %).

Beispiel 5: *N*-[2-(3,4-Dihydroxyphenyl)ethyl]-*N'*-hexylthioharnstoff

2-(3,4-Dihydroxyphenyl)ethylaminhydrochlorid (0,99 g, 5 mmol) und Triethylamin (1,01 g, 10 mmol) werden unter Stickstoff in $CHCl_3$ (15 ml) suspendiert und *n*-Hexylisothiocyanat (0,75 g, 5,25 mmol), gelöst in $CHCl_3$ (10 ml), zudosiert. Die Reaktionsmischung wird noch 18 h bei Raumtemperatur gerührt und anschließend mit 5 %iger Salzsäure gewaschen. Die organische Phase wird mit Wasser gewaschen, über $Na_2SO_4$ getrocknet, abfiltriert und das Filtrat bei 40°C/800 - 20 mbar eingedampft: 0,795 g (54 %); MS (APCI-): *m/z* = 295,59 (100 %, $[M-H]^-$), 590,94 (4 %, $[2M-H]^-$).

Beispiel 6: *N*-[2-(3,4-Dihydroxyphenyl)ethyl]-*N'*-hexylharnstoff

2-(3,4-Dihydroxyphenyl)ethylaminhydrochlorid (1 g, 5,3 mmol) und Triethylamin (2,04 g, 20 mmol) werden unter Stickstoff in $CHCl_3$ (25 ml) suspendiert und *n*-Hexylisocyanat (0,51 g, 4,06 mmol), gelöst in $CHCl_3$ (20 ml), innerhalb von 20 min zudosiert. Die Reaktionsmischung wird noch 18 h bei Raumtemperatur gerührt und anschließend mit 5 %iger Salzsäure gewaschen. Die organische Phase wird mit Wasser gewaschen, über $Na_2SO_4$ getrocknet, abfiltriert und das Filtrat bei 40°C/800 - 20 mbar eingedampft (1,5 g); das Filtrat wird an Kieselgel mit Chloroform/Methanol 10:1 chromatografiert. Ausbeute 0,38 g (26 %); $^1$H-NMR (400 MHz, $CD_3OD$): $\delta$ = 6,69 (1H, d, 8Hz), 6,64 (1H, d, 2 Hz), 6,52 (1H, dd, 8 Hz, 2 Hz), 3,26 (2H, t, 7 Hz), 3,07 (2H, t, 7 Hz), 2,62 (2H, t, 7 Hz), 1,45 (2H, m), 1,38-1,25 (8H, m), 0,90 (3H, t, 7 Hz) ppm; MS (APCI+): *m/z* = 281,30 (100 %, $[M+H]^+$), 560,92 (77 %, $[2M+H]^+$).

**Anwendungsbeispiele:**

Beispiel 7: Kosmetische "Öl in Wasser"-Emulsion

| Teil | Rohstoffname (Hersteller) | Chemische Bezeichnung | Gehalt in Gew.-% |
|---|---|---|---|
| A | Arlatone 983 S® (ICI) | Ether von Polyethylenglycol mit Glycerylmonostearat | 1,2 |
| | Brij 76® (ICI) | 3,6,9,12,15,18,21,24,27,30,33,36-Decaoxaoctatetracontan-1-ol | 1,2 |
| | Cutina MD® (Henkel) | Glycerylmonostearat | 3,5 |
| | Baysiloneöl M10® (GE Bayer) | Polydimethylsiloxan | 0,8 |
| | Eutanol G® (Henkel) | Octyldodecanol | 3,0 |
| | Paraffinöl 65 cp (Henry Lamotte) | Mineralöl | 8,0 |
| B | Wasser, dest. | | 50,35 |
| | Phenonip® (Nipa Laboratorien) | 2-Phenoxyethanol und 4-Hydroxybenzoesäuremethylester und 4-Hydroxybenzoesäure-ethylester und 4-Hydroxy-benzoesäurepropylester und 4-Hydroxybenzoesäurebutylester | 0,5 |
| | 1,2-Propylenglycol | | 2,0 |
| | Glycerin 99 % | | 3,0 |
| | Trilon® BD | Dinatrium-EDTA | 0,1 |
| | ***N*-[2-(3,4-Dihydroxyphenyl) ethyl]-*O*-methylurethan (Beispiel 1)** | | **0,1** |
| C | Wasser, dest. | | 25,0 |
| | Carbopol 2050® (B.F. Goodrich) | vernetztes Acrylsäure/$C_{10}$-$C_{30}$-Alkylacrylat-Polymer | 0,4 |
| | wässrige Natriumhydroxidlsg., 10 % | | 0,85 |

Teil A wurde gemischt und auf 80°C erhitzt. Teil B wurde gemischt und auf 90°C erhitzt und unter Rühren zu Teil A gegeben. Für Teil C wurde Carbopol in Wasser sorgfältig dispergiert und mit Natronlauge neutralisiert (pH 5,4). Teil C wurde dann bei 60°C zur Mischung aus den Teilen A und B gegeben.

Beispiel 8: Kosmetische Butylenglycol-Lösung

| Rohstoffname | Gehalt in Gew.-% |
|---|---|
| 1,3-Butylenglycol | 99,9 |
| ***N*-[2-(3,4-Dihydroxyphenyl)ethyl]-*O*-methylurethan (Beispiel 1)** | **0,1** |

Beispiel 9: Kosmetische "Wasser in Öl"-Sonnenschutzemulsion mit UVA/B-Breitbandschutz

| Teil | Rohstoffname (Hersteller) | Chemische Bezeichnung | Gehalt in Gew.-% |
|---|---|---|---|
| A | Dehymuls PGPH® (Henkel) | Polyglyceryl-2 Dipolyhydroxystearat | 3,0 |
| | Monomuls 90-O 18® (Henkel) | Glyceryloleat | 1,0 |
| | Permulgin 2550® (Koster Keunen Holland) | Bienenwachs | 1,0 |
| | Myritol 318® (Henkel) | Capryl-/Capronsäuretriglyceride | 6,0 |
| | Witconol TN® (Witco) | $C_{12}$-$C_{15}$-Alkylbenzoat | 6,0 |
| | Cetiol SN® (Henkel) | Cetyl- und Stearylisononanoat | 5,0 |
| | Copherol 1250® (Henkel) | Tocopherolacetat | 1,0 |
| | Solbrol P® (Bayer) | 4-Hydroxybenzoesäurepropyl-ester | 0,1 |
| | Neo Heliopan® AV (Haarmann & Reimer) | 2-Ethylhexyl-p-methoxy-cinnamat | 4,0 |
| | Neo Heliopan® E 1000 (Haarmann & Reimer) | Isoamyl-p-methoxycinnamat | 4,0 |
| | Neo Heliopan® MBC (Haarmann & Reimer) | 3-(4-Methylbenzyliden)-dl-campher | 2,0 |
| | Neo Heliopan® OS (Haarmann & Reimer) | 2-Ethylhexylsalicylat | 3,0 |
| | Octyltriazon | | 1,0 |
| | Zinkoxid neutral (Haarmann & Reimer) | | 7,0 |
| B | Wasser, dest. | | 40 |
| | Phenoxyethanol | | 0,7 |
| | Solbrol® M (Bayer) | 4-Hydroxybenzoesäuremethyl-ester | 0,2 |
| | Glycerin 99 % | | 4,0 |
| | Neo Heliopan® Hydro (Haarmann & Reimer), 15 % als Natriumsalz | 2-Phenylbenzimidazol-5-sulfonsäure | 10,0 |
| | Benzophenon-4 | | 0,5 |
| | ***N*-[2-(3,4-Dihydroxyphenyl)ethyl]-*O*-methylurethan (Beispiel 1)** | | **0,1** |
| C | Parfümöl | | 0,3 |
| | Bisabolol | | 0,1 |

Für Teil A wurden alle Substanzen bis auf das Zinkoxid auf 85°C erhitzt und das Zinkoxid in der Mischung sorgfältig dispergiert. Die Komponenten des Teils B wurden gemischt, auf 85°C erhitzt und unter Rühren zu Teil A gegeben. Zu der Mischung aus den Teilen A und B wurde Teil C zugegeben und anschließend die Mischung mit einem Dispergierwerkzeug homogenisiert.

Beispiel 10: Zubereitung eines Speiseöls für Frittierzwecke

| Rohstoffname | Gehalt in Gew.-% |
|---|---|
| Sojaöl, raffiniert | 99,95 |
| ***N*-[2-(3,4-Dihydroxyphenyl)ethyl]-*N'*-hexylharnstoff (Beispiel 6)** | **0,05** |

## Experimente

Beispiel 11: Aktivität als Radikalfänger

Die Aktivität der beispielhaften Verbindungen als Radikalfänger wurde mit der herkömmlicher Radikalfänger verglichen. Dabei wurde der DPPH-(1,1-Diphenyl-2-picryl-hydrazyl)-Test zur Beseitigung von Radikalen angewendet (Lebensm.-Wiss. u. Technol., 1995, Bd. 28, S. 25 bis 30).

DPPH wurde in Methanol zu einer Konzentration von 100 µmol/l gelöst. Eine Reihe von Verdünnungen der beispielhaften Verbindungen, Vitamin C, α-Tocopherol und 3,5-Di-tert.-butyl-4-hydroxytoluol wurden in Methanol hergestellt. Methanol diente als Kontrolle. 2500 µl der DPPH-Lösung wurden mit 500 µl einer jeden Testlösung gemischt und die Abnahme der Absorption bei 515 nm solange abgelesen, bis die Abnahme kleiner 2 % pro Stunde war. Die Aktivität der Testsubstanzen als Radikalfänger wurde nach folgender Gleichung berechnet:

$$\text{Aktiv. als Radikalfänger (\%)} = 100 - \text{(Absorption der Testverbindungen)/(Absorption der Kontrolle)} \times 100.$$

Aus der Aktivität als Radikalfänger (%) in einer Reihe von Verdünnungen von Testverbindungen wurde für jede Testverbindung die effektive relative Konzentration $EC_{50}$ (bezogen auf die anfangs vorhandene Konzentration an DPPH, EC = c (Testverbindung)/c(DPPH)) einer Testverbindung berechnet, bei der das Radikal DPPH um 50 % beseitigt wurde. Je kleiner die $EC_{50}$-Werte sind, desto besser ist die radikalfangende Wirkung.

Die Ergebnisse sind in Tabelle 1 dargestellt:

Tabelle 1

| **Testverbindung (Beispielnummer)** | **$EC_{50}$ / (mol/mol)** |
|---|---|
| *N*-[2-(3,4-Dihydroxyphenyl)ethyl]-*O*-methylurethan (1) | 0,27 |
| *N*-[2-(3,4-Dihydroxyphenyl)ethyl]-*O*-[(1*R*,3*R*,4*S*)-menthyl]urethan (2) | 0,35 |
| *N*-[2-(3,4-Dihydroxyphenyl)ethyl]-*O*-hexylurethan (3) | 0,24 |
| *N*-[2-(3,4-Dihydroxyphenyl)ethyl]-*O*-(2-ethylhexyl)urethan (4) | 0,25 |
| *N*-[2-(3,4-Dihydroyxphenyl)ethyl]-*N'*-hexylharnstoff (6) | 0,20 |
| Vitamin C | 0,27 |
| α-Tocopherol | 0,25 |
| 3,5-Di-tert.-butyl-4-hydroxytoluol | 0,24 |

Beispiel 12: Aktivität als Antioxidantien

Die Aktivität der beispielhaften Verbindungen als Antioxidantien wurde mit der herkömmlicher Antioxidantien verglichen. Als Testsystem wurde die beschleunigte Autoxidation von Lipiden durch Luft mit oder ohne Antioxidans mit Hilfe der Rancimat-Apparatur angewendet (Deutsche Lebensmittel-Rundschau 1974, Bd. 70, S. 57 bis 65) (Rancimat ist ein eingetragenes Warenzeichen der Metrohm AG, Herisau, Schweiz).

Die beispielhaften Verbindungen, Vitamin C, α-Tocopherol und 3,5-Di-tert.-butyl-4-hydroxytoluol wurden in Methanol oder Aceton gelöst und von der jeweiligen Testlösung 100 µl zu einer vorbereiteten Ölprobe von 3 g gegeben. In eine Kontrollprobe wurde nur Lösungsmittel gegeben. Durch die aufgeheizte, die Testlösung enthaltende Ölprobe wurde ein konstanter, trockener Luftstrom (20 l/h) geblasen und die flüchtigen Oxidationsprodukte (vorwiegend kurzkettige Fettsäuren wie Ameisen- oder Essigsäure) in einer Vorlage mit Wasser gesammelt. Die Leitfähigkeit dieser wässrigen Lösung wurde kontinuierlich gemessen und dokumentiert. Die Oxidation von (ungesättigten) Fetten verläuft dabei eine Zeitlang nur sehr langsam und steigt dann plötzlich stark an. Die Zeit bis zum Anstieg wird als Induktionsperiode (IP) bezeichnet.

Nach der folgenden Gleichung wurde der antioxidative Index (AOI) erhalten:

$$AOI = IP_{(\text{mit Testlösung})} / IP_{(\text{Kontrollprobe})}.$$

Je größer der AOI-Wert ist, desto höher ist die antioxidative Wirkung.

Die Ergebnisse für das Experiment bei 100°C in Sojaöl, dass über Alumina Typ N gereinigt wurde, sind in Tabelle 2 dargestellt:

Tabelle 2

| **Testverbindung (Beispielnummer)** | **AOI in Sojaöl bei 100°C mit 0,05% Testsubstanz** |
|---|---|
| *N*-[2-(3,4-Dihydroxyphenyl)ethyl]-*O*-methylurethan (1) | 15,3 |
| *N*-[2-(3,4-Dihydroxyphenyl)ethyl]-*O*-[(1*R*,3*R*,4*S*)-menthyl] urethan (2) | 7,1 |
| *N*-[2-(3,4-Dihydroxyphenyl)ethyl]-*O*-hexylurethan (3) | 9,8 |
| *N*-[2-(3,4-Dihydroxyphenyl)ethyl]-*O*-(2-ethylhexyl)urethan (4) | 7,6 |
| *N*-[2-(3,4-Dihydroxyphenyl)ethyl]-*N'*-hexylthioharnstoff (5) | 14,4 |
| *N*-[2-(3,4-Dihydroyxphenyl)ethyl]-*N'*-hexylharnstoff (6) | 26 |
| Vitamin C | 1,2 |
| α-Tocopherol | 5,1 |
| 3,5-Di-tert.-butyl-4-hydroxytoluol | 4,8 |

Die Ergebnisse für das Experiment bei 80°C in Squalen, dass über Alumina Typ N gereinigt und mit 1 ppm α-Tocopherol stabilisiert wurde, sind in Tabelle 3 dargestellt:

Tabelle 3

| **Testverbindung (Beispielnummer)** | **AOI in Squalen bei 80°C mit 0,005% Test substanz** |
|---|---|
| *N*-[2-(3,4-Dihydroxyphenyl)ethyl]-*O*-methylurethan (1) | 76 |
| *N*-[2-(3,4-Dihydroxyphenyl)ethyl]-*O*-[(1*R*,3*R*,4*S*)-menthyl] urethan (2) | 25 |
| *N*-[2-(3,4-Dihydroxyphenyl)ethyl]-*O*-hexylurethan (3) | 46 |
| *N*-[2-(3,4-Dihydroxyphenyl)ethyl]-*O*-(2-ethylhexyl)urethan (4) | 50 |
| *N*-[2-(3,4-Dihydroxyphenyl)ethyl]-*N'*-hexylthioharnstoff (5) | 59 |
| *N*-[2-(3,4-Dihydroxyphenyl)ethyl]-*N'*-hexylharnstoff (6) | 60 |
| Vitamin C | 0,7 |
| α-Tocopherol | 39 |
| 3,5-Di-tert.-butyl-4-hydroxytoluol | 38 |

Wie aus den Experimenten in den Beispielen 11 und 12 ersichtlich, sind die erfindungsgemäßen Verbindungen gleich gute Radikalfänger und sogar wesentlich bessere Antioxidantien im Vergleich zu den Standardantioxidantien Vitamin C, α-Tocopherol oder 3,5-Di-tert.-butyl-4-hydroxytoluol.

## Patentansprüche

1. Verwendung von 2-(3,4-Dihydroxyphenyl)ethyl-substituierten Kohlensäurederivaten der allgemeinen Formel I,

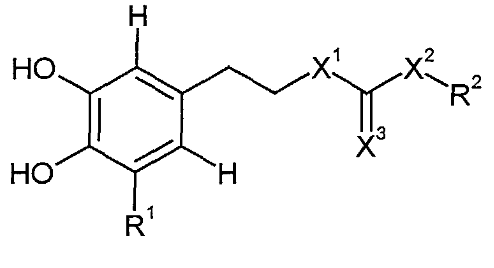

(I)

wobei

$R^1$ ein Wasserstoffatom, eine Niederalkyl-, Niederalkenyl-, 1-Oxoniederalkyl- oder 1-Oxoniederalkenylgruppe oder eine Gruppe -O-$R^3$ darstellt, in der $R^3$ ein Wasserstoffatom, eine Niederalkyl-, Niederalkenyl-, 1-Oxoniederalkyl- oder 1-Oxoniederalkenylgruppe bedeutet,

und

$X^1$, $X^2$ und $X^3$ unabhängig voneinander Sauerstoff, Schwefel oder NH darstellen,

und

$R^2$ eine verzweigte oder unverzweigte, cyclische oder gestreckte Alkylgruppe mit 1 bis 22 Kohlenstoffatomen oder eine verzweigte oder unverzweigte, cyclische oder gestreckte Alkenylgruppe mit 2 bis 22 Kohlenstoffatomen, wobei eins oder mehrere der Kohlenstoffatome durch Sauerstoffatome ersetzt werden können, oder eine kernsubstituierte Arylalkylgruppe mit 7 bis 15 Kohlenstoffatomen bedeutet,

als Antioxidantien oder Radikalfänger.

**2.** Verwendung von 2-(3,4-Dihydroxyphenyl)ethyl-substituierten Kohlensäurederivaten der allgemeinen Formel I nach Anspruch 1,
wobei

$R^1$ ein Wasserstoffatom, eine Niederalkylgruppe mit 1 bis 5 Kohlenstoffatomen oder eine Gruppe -O-$R^3$ darstellt, in der $R^3$ ein Wasserstoffatom oder eine Methylgruppe bedeutet,

und

$X^1$, $X^2$ und $X^3$ unabhängig voneinander Sauerstoff, Schwefel oder NH darstellen,

und

$R^2$ eine verzweigte oder unverzweigte, cyclische oder gestreckte Alkylgruppe mit 1 bis 18 Kohlenstoffatomen oder eine verzweigte oder unverzweigte, cyclische oder gestreckte Alkenylgruppe mit 2 bis 20 Kohlenstoffatomen oder einen kernsubstituierten Benzyl-, kernsubstituierten 2-Phenylethyl oder kernsubstituierten 1-Phenylethylrest darstellt,

als Antioxidantien oder Radikalfänger.

**3.** Verwendung von *N*-[2-(3,4-Dihydroxyphenyl)ethyl]-*O*-methylurethan, *N*-[2-(3,4-Dihydroxyphenyl)ethyl]-*O*-[(1*R*,3*R*,4*S*)-menthyl]urethan, *N*-[2-(3,4-Dihydroxyphenyl)ethyl]-*O*-hexylurethan, *N*-[2-(3,4-Dihydroxyphenyl)ethyl]-*O*-(2-ethylhexyl)urethan, *N*-[2-(3,4-Dihydroxyphenyl)ethyl]-*N'*-hexylthioharnstoff und *N*-[2-(3,4-Dihydroxyphenyl)ethyl]-*N'*-hexylharnstoff als Antioxidantien oder Radikalfänger.

**4.** Verwendung von 2-(3,4-Dihydroxyphenyl)ethyl-substituierten Kohlensäurederivaten der allgemeinen Formel I nach Anspruch 1 zum Schutze kosmetischer, dermatologischer sowie der Ernährung oder dem Genuss dienender Zubereitungen gegen Oxidation oder Photooxidation.

**5.** Kosmetische und dermatologische Zubereitungen, enthaltend die in den Ansprüchen 1 bis 3 genannten 2-(3,4-Dihydroxyphenyl)ethyl-substituierten Kohlensäurederivate.

**6.** Der Ernährung oder dem Genuss dienende Zubereitungen, enthaltend die in den Ansprüchen 1 bis 3 genannten 2-(3,4-Dihydroxyphenyl)ethyl-substituierten Kohlensäurederivate.

**7.** 2-(3,4-Dihydroxyphenyl)ethyl-substituierte Kohlensäurederivate der allgemeinen Formel (II)

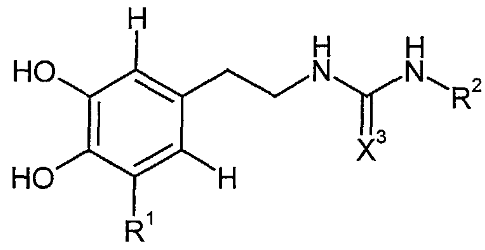

(II)

wobei

$R^1$ ein Wasserstoffatom, eine Niederalkyl-, Niederalkenyl-, 1-Oxoniederalkyl- oder 1-Oxoniederalkenylgruppe oder eine Gruppe -O-$R^3$ darstellt, in der $R^3$ ein Wasserstoffatom, eine Niederalkyl-, Niederalkenyl-, 1-Oxoniederalkyl- oder 1-Oxoniederalkenylgruppe bedeutet,

und

$X^3$ Sauerstoff, Schwefel oder NH darstellt,

und

$R^2$ eine verzweigte oder unverzweigte, cyclische oder gestreckte Alkylgruppe mit 1 bis 22 Kohlenstoffatomen oder eine verzweigte oder unverzweigte, cyclische oder gestreckte Alkenylgruppe mit 2 bis 22 Kohlenstoffatomen darstellt.

**8.** 2-(3,4-Dihydroxyphenyl)ethyl-substituierte Kohlensäurederivate nach Anspruch 7 wobei

$R^1$ ein Wasserstoffatom, eine Niederalkylgruppe mit 1 bis 5 Kohlenstoffatomen oder eine Gruppe -O-$R^3$ darstellt, in der $R^3$ ein Wasserstoffatom oder eine Methylgruppe bedeutet,

und

$X^3$ Sauerstoff oder Schwefel darstellt,

und

$R^2$ eine verzweigte oder unverzweigte, cyclische oder gestreckte Alkylgruppe mit 1 bis 18 Kohlenstoffatomen oder eine verzweigte oder unverzweigte, cyclische oder gestreckte Alkenylgruppe mit 2 bis 20 Kohlenstoffatomen darstellt.

**9.** Verfahren zur Herstellung der 2-(3,4-Dihydroxyphenyl)ethyl-substituierten Kohlensäurederivate nach Anspruch 7, **dadurch gekennzeichnet, dass** man 2-(3,4-Dihydroxyphenyl)ethylamine der allgemeinen Formel (III)

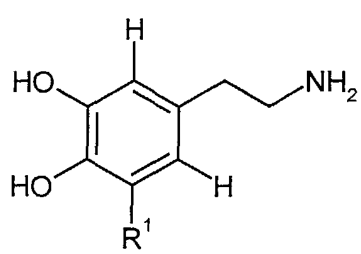

(III)

wobei $R^1$ die in Anspruch 7 aufgeführte Bedeutung hat,
oder deren kationischen Salze

mit einem Heterokumulen der allgemeinen Formel (IV),

$$X^4{=}C{=}N{-}R^2 \qquad \text{(IV)}$$

wobei

$X^4$ ein Sauerstoffatom oder ein Schwefelatom darstellt

und

$R^2$ die in Anspruch 7 genannte Bedeutung hat,

oder
mit Phosgen, Thiophosgen oder Triphosgen mit oder ohne Lösungsmittel und gegebenenfalls unter Beimengung einer Hilfsbase und anschließend entweder direkt nach Aufreinigung oder ohne Aufreinigung mit einer Verbindung der allgemeinen Formel (V),

$$Z{-}R^2 \qquad \text{(V)}$$

wobei Z eine Gruppe -O-H, -S-H, $-NH_2$ oder $-(NH_3)^+$ darstellt
und

$R^2$ die oben genannte Bedeutung hat

mit oder ohne Lösungsmittel und gegebenenfalls unter Beimengung einer Hilfsbase umsetzt.

**Europäisches Patentamt**

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung: EP 02 01 6986

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.7) |
|---|---|---|---|
| P,X | WO 01 98235 A (LANGNER ROLAND ;LEY JAKOB PETER (DE); HAARMANN & REIMER GMBH (DE);) 27. Dezember 2001 (2001-12-27) *das ganze Dokument* --- | 1-9 | A61K47/16<br>A61K47/08<br>A61K47/20<br>A61K7/00<br>C11B5/00<br>C07C335/12<br>C07C275/24<br>C07C279/08 |
| A | EP 0 900 781 A (HAARMANN & REIMER GMBH) 10. März 1999 (1999-03-10) *das ganze Dokument* --- | 1-9 | |
| D,A | DATABASE WPI Section Ch, Week 198531 Derwent Publications Ltd., London, GB; Class B05, AN 1985-187167 XP002224476 & JP 60 115558 A (IWAZAKI SEIYAKU KK), 22. Juni 1985 (1985-06-22) * Zusammenfassung * --- -/-- | 1-9 | |

RECHERCHIERTE SACHGEBIETE (Int.Cl.7)

C07C
A61K
C11B

## UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

| Recherchenort | Abschlußdatum der Recherche | Prüfer |
|---|---|---|
| MÜNCHEN | 12. Dezember 2002 | Österle, C |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03 82 (P04C09)

Europäisches Patentamt

**UNVOLLSTÄNDIGE RECHERCHE ERGÄNZUNGSBLATT C**

Nummer der Anmeldung
EP 02 01 6986

Unter die Verwendungsansprüche 1-4 fällt auch die Verwendung der Verbindungen zur Behandlung von Tieren, bzw. Menschen (siehe z.B. S. 7 der Beschreibung).
Obwohl die Ansprüche 1-4 sich also auch auf ein Verfahren zur Behandlung des menschlichen/tierischen Körpers beziehen (Artikel 52(4) EPÜ), wurde die Recherche durchgeführt und gründete sich auf die angeführten Wirkungen der Verbindung.

Europäisches Patentamt

# EUROPÄISCHER TEILRECHERCHENBERICHT

Nummer der Anmeldung
EP 02 01 6986

| EINSCHLÄGIGE DOKUMENTE | | | KLASSIFIKATION DER ANMELDUNG (Int.Cl.7) |
|---|---|---|---|
| Kategorie | Kennzeichnung des Dokuments mit Angabe, soweit erforderlich der maßgeblichen Teile | Betrifft Anspruch | |
| D,A | FRANK W. FOWLER ET AL: "Design of molecular solids: utility of the hydroxyl functionality as a predictable design element" NEW J. CHEM., 1998, Seiten 129-135, XP009002774 *compound 6* ----- | 7 | |
| | | | RECHERCHIERTE SACHGEBIETE (Int.Cl.7) |

EPO FORM 1503 03.82 (P04C12)

## ANHANG ZUM EUROPÄISCHEN RECHERCHENBERICHT ÜBER DIE EUROPÄISCHE PATENTANMELDUNG NR. EP 02 01 6986

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-12-2002

| Im Recherchenbericht angeführtes Patentdokument | Datum der Veröffentlichung | Mitglied(er) der Patentfamilie | Datum der Veröffentlichung |
|---|---|---|---|
| WO 0198235 A | 27-12-2001 | DE 10030891 A1 | 03-01-2002 |
| | | AU 7567701 A | 02-01-2002 |
| | | WO 0198235 A2 | 27-12-2001 |
| EP 0900781 A | 10-03-1999 | DE 19737327 A1 | 04-03-1999 |
| | | AU 738508 B2 | 20-09-2001 |
| | | AU 8189898 A | 11-03-1999 |
| | | DE 59803410 D1 | 25-04-2002 |
| | | EP 0900781 A2 | 10-03-1999 |
| | | JP 11228514 A | 24-08-1999 |
| | | US 6117365 A | 12-09-2000 |
| JP 60115558 A | 22-06-1985 | KEINE | |

EPO FORM P0461

Für nähere Einzelheiten zu diesem Anhang : siehe Amtsblatt des Europäischen Patentamts, Nr.12/82